# EUROPEAN PATENT APPLICATION

(11) **EP 4 257 096 A1**
(43) Date of publication of application: **11.10.2023**
(21) Application number: 22167176.1
(22) Date of filing: 07.04.2022
(51) Int. Cl.: A61F 5/01, A61F 5/34

(54) **ORTHOPAEDIC ORTHOSIS FOR UPPER LIMB**

(71) Applicant: Medirpintic Spolka Z Organiczona Odpowiedzialnoscia, 35-005 Rzeszow (PL)
(72) Inventor: Zakrecki, Andrzej, 31-271 Krakow (PL); Kostuj, Marcin, 36-100 Kolbuszowa (PL); Machaj, Aleksander, 39-400 Tarnobrzeg (PL)
(74) Representative: AOMB Polska Sp. z.o.o.

(57) **Abstract**

Orthosis for upper limb, comprising a body made of a first part (1) and a second part (3) adapted for being joined together around the limb to be stabilized, the first part and the second part being adjustable relative to each other; connection means for joining together the first part and the second part; and an adjustment system for adjusting the position of the first part and the second part relative to each other, wherein the adjustment system comprises means for adjusting the position of the first part and the second part in the radial direction, including guiding surfaces (13) disposed on the first part and adjustment locks (12) disposed on the inside of the first part, extending in the axial direction of the orthosis and determining the minimum cross-section of the orthosis in the radial direction, wherein the guiding surfaces are sections of the first part extending between its longitudinal edges and these adjustment locks, whereby the guiding surfaces are slipped over the complementary lateral surfaces of the second part while the first part and the second part are being joined together.

## Description

### Technical field

The present invention relates to the field of orthopaedic devices for immobilizing bone or joint injuries. More specifically, the invention relates to the field of orthoses for immobilization or treatment of forearm injuries.

### Background art

Various instruments and structures supporting the treatment of fractures and injuries of bones and joints are known in the art, such as splints, orthopaedic plaster, orthoses or orthopaedic rails. The forearm orthoses known at present are mostly structures made of soft materials, materials used in the 3D printing technology (e.g. PA12 polyamide, PETG), and composite materials. Their main disadvantage is that they do not allow for proper fit and stiffness to be maintained during treating the forearm injuries, which creates the risk of complications and does not provide a sufficiently high guarantee of correct treatment effect.

In case of known standard structures, such as a plaster cast, the time required to apply this cast is very long and can be as long as about one hour, which is also influenced by the need to check the initial drying of the plaster cast so that it does not pinch and is adapted to the patient's needs. This cast, once fitted to the patient's limb, cannot be adapted to the limb in line with progressing treatment, during which the swelling can be absorbed or increased. As a result, the required rigidity is not provided, which creates the risk of complications and does not provide a high guarantee of the correct union of bones. As the arm can move sideways in the plaster cast space and thus lead to the bone chip displacement, a decreasing or strongly growing swelling can lead to various complications, e.g. Sudeck syndrome. The fracture can also be displaced due to the resorption of swelling.

More advanced solutions of 3D printed two-part orthoses, provided with various means allowing both parts to be joined together and fitted to the patient's limb, are also known. Examples of means of connection include braces, straps, Velcros, or cables with the adjusting mechanism. Also known are methods for manufacturing two-part orthoses that use a 3D scanner to create a 3D virtual limb model, which then is used as a base for the system to create an appropriate orthosis arrangement. The literature also includes various modular solutions that can be added to the orthosis as accessories. These include, for example, stabilizing elements or clockworks.

Known orthoses are fabricated based on a 3D scan that needs to be performed by a technician who scans the forearm and on this basis a 3D printed orthosis design is developed. The patient must visit the orthopaedic practice where the produced orthosis is put on him/her. Athletes after trauma are treated in a model of intensive and very costly rehabilitation, but such rehabilitation is not available to the ordinary patient because of the huge costs and the need to involve many people in the care of one patient. However, the rapid return to full physical fitness is very important for both people professionally involved in sports, as well as for people working in other professions.

Below, selected solutions known from patent literature are presented.

EP 2400935 B1 discloses a process for creating a three-dimensional orthopaedic product using a composite material. The orthopaedic product consists of one part, in which appropriate part of the body or limb is inserted, and it is stiffened with a cable and a knob which help in adapting the product to the correct size. The cables are secured in appropriate holes in the guiding mechanism, which is not an integral part of the orthopaedic product, and a roller mechanism is used to mechanically tighten and lock the tensioned cable.

EP 3138539 B1 discloses the design of a mechanism fastening the forearm orthosis with a Velcro system and a cable, while EP 3431055 B1 discloses the design of a mechanism fastening the forearm orthosis by a system of elastic rubbers. EP 2744459 B1, in turn, discloses the design of an orthosis with a clamp system comprising a hinge and braces.

WO2019057811A1 relates to an orthosis for bending and/or stretching of at least one finger of the patient's hand, having drive means attached to the splint and connected to at least one finger segment, and comprising a plurality of members connected via hinges. WO2020188036A1, in turn, relates to a hand finger orthosis including at least three articulated joints for finger stabilization.

EP3638161A1 relates to a splint for treatment of forearm bone fractures, which has two parts adapted to each other, and means of connection to join said parts together so that they are immobilized. The two matching parts are the palmar part of the forearm and the dorsal part of the forearm, which exert pressure on the broken bones and are held together in a fixed manner, and additionally, the means of connection enable the position of the two parts to be adjusted relative to each other.

DE102016116388A1 discloses an orthosis consisting of at least one finger segment, a hand segment, and a forearm segment, pivotally connected to each other. The said orthosis includes additionally a tensioning device, consisting of a knob and a tensioning cable, which adjust the inclination angle of the fingers and the hand relative to the forearm. In addition, all said movable segments of the orthosis have eyelets for tightening straps, which can be used to adapt the diameters of said segments to the diameter of the limb to be stiffened.

WO2021156894A1 discloses a two-part orthosis produced with a 3D printer. The two parts of the orthosis are fitted to each other and adapted to be connected to each other by a Velcro. The orthosis includes also an arrangement (grid) of ventilation holes, and is fabricated with an incremental technique based on a CAD model and the finite element method (FEA).

### Summary of the invention

In response to the real need to improve the treatment quality of the upper limb injuries, a modular forearm orthosis with adjustable clamping pressure is developed. The developed orthosis overcomes the problems known from the prior art.

An orthopaedic orthosis, in particular for upper limb, according to the invention, comprises a body made of a first part and a second part adapted for being joined together around the limb to be stabilized, the first part and the second part being adjustable relative to each other; connecting means for joining together the first part and the second part; and an adjustment system for adjusting the position of the first part and the second part relative to each other, wherein the adjustment system comprises means for adjusting the position of the first part and the second part in the radial direction, said means include guiding surfaces disposed on the first part and adjustment locks, wherein the adjustment locks are disposed on the inside of the first part, extend in the axial direction of the orthosis and determine the minimum cross-section of the orthosis in the radial direction, and wherein the guiding surfaces constitute sections of the first part, extending between its longitudinal edges and these adjustment locks, whereby the guiding surfaces are slipped over the complementary lateral surfaces of the second part while the first part and the second part are being joined together.

The guiding surfaces can be from 1 cm to 10 cm wide, and even more preferably from 3 cm to 8 cm.

In one of the embodiments, the means for adjusting the position of the first part and the second part comprise additionally a cable attached to a knob disposed on the first part, and guides disposed alternately on the left and the right side of the first part on its lateral surfaces along both longitudinal edges, wherein the cable forms a closed loop and is passed through the guides so that it passes alternately through the guides on the left and the right side of the first part, so that the individual cable sections intersect in the area of the second part.

In one of the preferred embodiments, the second part of the body comprises at least one socket for receiving one or more finger clips. The clips, such as a thumb clip or a clip for the other fingers, serve as protection and stabilize finger injuries.

The adjustment locks are preferably in the form of continuous bulging or spot protrusions extending along the longitudinal edge and at a certain distance from it.

In one of the embodiments, the first part and the second part are fitted with lining material disposed on their inner side.

The orthosis can also be fitted with a safety mechanism that protects the knob from misadjustment, preferably in the form of a latch.

In a preferred embodiment, the orthosis has six guides, three on each of the lateral surfaces of the first part. The guiding surfaces are preferably 15-45%, and even more preferably 25% thinner than the central section of the first part, extending between the adjustment locks.

In one of the alternative embodiments, the orthosis has spaces for installation of cushions, wherein the first cushion space is located in the first part, the second cushion space is located in the second part, near the hand area, and the third cushion space is located in the second part, near the wrist area.

In yet another embodiment, the orthosis has a cushion inflation device, comprising a pump made of flexible material and connected tightly by tubing with each of the cushions, wherein each of the cushions is fitted with a valve. The cushion inflation device can also be fitted with a one-way valve to prevent the air from flowing back from the cushions.

### Advantageous effects of the invention

By providing a forearm orthosis with modular design, fitted with an adjustment system with guides, the present invention enables quick treatment of both stable and unstable injuries, depending on the patient's anatomy. The orthosis can be adapted to the type of injury and can be applied within a few minutes only. The structure of the orthosis ensures appropriate stiffness of the forearm and allows the clamping pressure to the limb surface to be adjusted so that it is possible to maintain an appropriate degree of immobilization in line with gradual swelling resorption. The clamping pressure adjustment system of the orthosis ensures appropriate stiffness during the healing process and allows for adjustment in the first days after the injury, which ensures greater certainty of the correct bone union and allows to reduce the risk of complications such as displacement of fracture due to swelling resorption or formation of a space in the cast, e.g. Sudeck syndrome. The orthosis can be easily taken off and put on by the patient with the help of an orthopaedist or therapist or, in some cases, even by the patient himself/herself. This allows the patient to consult his/her orthopaedist remotely, for example via online consultation, thus saving time for travelling or waiting in the queue, and the orthopaedist can recommend specific exercises without meeting the patient in the office. Hence, the orthosis according to the invention enables also to start an early, independent rehabilitation that would allow partial muscle decay (as is the case with the use of traditional plaster) to be avoided, so that the patient could relatively quickly return to the full physical fitness. In addition, the ability to take off the orthosis independently as well as the orthosis structure make it easier to maintain hygiene in the injury area, as well as to relieve itching of the skin under the dressing.

### Description of the drawings

The invention will be now explained in more detail in a preferred embodiment with reference to the accompanying figures, wherein:
Fig. 1 shows a two-part forearm orthosis in the assembled state in the top view, from the upper edge side of the forearm;
Fig. 2 shows a two-piece forearm orthosis in the folded state in the bottom view, from the palmar side of the forearm;
Fig. 3a shows the first part of the orthosis from the forearm opening side;
Fig. 3b shows the first part of the first orthosis from the left side (thumb side);
Fig. 3c shows the first part of the orthosis from the right side (small finger side);
Fig. 4a shows the second part of the orthosis in the form of an orthopaedic splint in the outside view;
Fig. 4b shows the second part of the orthosis in the form of an orthopaedic splint in the side view;
Fig. 4c shows the second part of the orthosis in the form of an orthopaedic splint in the inside view;
Fig. 5 shows the orthosis in the assembled state in the front view, from the hand opening side;
Fig. 6a shows the orthosis in the assembled state in the view from the second part (from the bottom);
Fig. 6b shows the orthosis in the assembled state in the side view;
Fig. 7 shows the orthosis in the assembled state with the thumb clip attached and with the sockets for clips for the other fingers;
Fig. 8 shows clips for the other fingers;
Fig. 9 shows the orthosis in the form of an orthopaedic splint in the assembled state with installed two clips for the other fingers;
Fig. 10a shows the second part of the orthosis in the form of an orthopaedic splint with installed one clip for the small and ring fingers;
Fig. 10b shows the second part of the orthosis in the form of an orthopaedic splint with installed one clip for the central and index fingers;
Fig. 10c shows the second part of the orthosis in the form of an orthopaedic splint with installed two clips for the other fingers - small, ring, as well as central and index fingers;
Fig. 11 shows the first part of the orthosis with the cushion space and the inflation device;
Fig. 12 shows the second part of the orthosis with spaces for cushions.

### Detailed description of the preferred embodiment

An orthosis according to the invention is intended to be used generally to assist in the treatment of fractures and injuries of bones and joints of the forearm. More specifically, it can be used to treat scaphoid fractures and provides basic immobilization of the metacarpophalangeal (MCP) and carpometacarpal (CMC) joints of the thumb, as well as additional immobilization of the radiocarpal and radioulnar joints. In addition, it can be used to treat various types of wrist injuries, simple fractures, dislocations and overloads (for immobilization), tendinitis, cumulative trauma disorders, as well as to treat pain and as a post surgery stabilizer. The modular design of the forearm orthosis, fitted with an adjustment system with guides, allows the physician to quickly dress up the arm, wrist and hand injuries, depending on the type of injury and the patient's anatomy, i.e. to adapt the orthosis to the type of injury and the anatomy of the patient.

Basically, the orthosis according to the invention has a two-part shell (body), preferably printed with a 3D printer, clipping devices (smaller, e.g. for fingers, or larger - in the form of a stabilizer with a clockwork to immobilize the elbow joint), means for connection and adjustment with a cable and knob mechanism - independently or in combination with Velcro, and a system of guides that allows the orthosis to be fitted to the shape of the limb as the treatment progresses. This design provides adequate stiffness for the forearm to stabilize it and provides the ability to adjust the clamping pressure to the limb surface so that after gradual desorption the necessary degree of immobilization is maintained. The orthosis can be fabricated in a variety of shapes for different types of injuries and it only takes a few minutes to put it on.

The orthosis is made of high-rigidity materials and can be further adapted by changing the thickness of the material used to fabricate it. The size of the orthosis and how it fits the body can be adjusted with an adjustment system with a guide-based mechanism. Due to this design the orthosis has an adjustment system throughout its cross-section, allowing it to be effectively clamped to the patient's forearm circumference over the entire length of the orthosis. In addition, the orthosis has a 2-stage clamping pressure control system for the forearm. The system comprises two parts of the orthosis and an adjustable cable. The adjustment is performed by tightening the cable from the wrist to the middle of the forearm, thus ensuring that the two parts of the orthosis are evenly clamped to the forearm on each side. The second stage of the orthosis adjustment is related to the control of clamping pressure on the metacarpal circumference with Velcro.

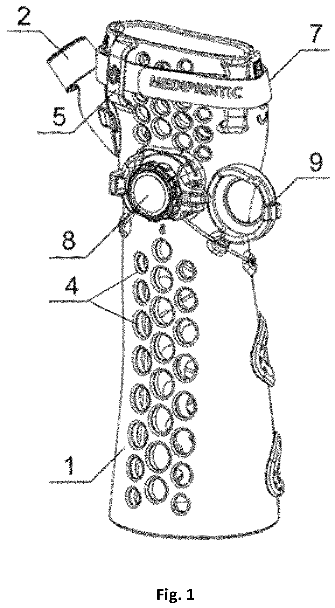

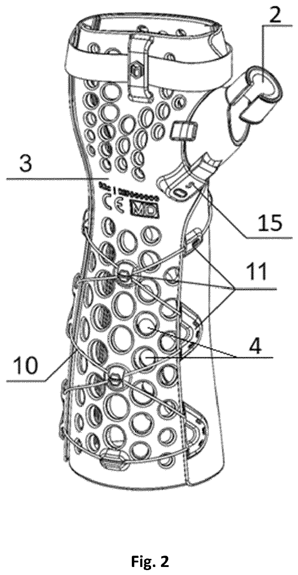

The object of the invention is shown in the embodiment in Fig. 1 and Fig. 2, which present a two-part forearm orthosis with an adjustment system with guides, for treating patients with forearm injuries, in the top and bottom views, respectively. The orthosis body is preferably fabricated by 3D printing, after prior development of a CAD model. For those skilled in the art it will be obvious that the body can be fabricated with plastics with any other known method, e.g. by injection moulding, vacuum casting or thermoforming. The orthosis has two main parts of the body, the first part 1 and the second part 3, these parts being adapted to be joined together so that when applied to the upper limb, they embrace it. The first part 1 is adapted to adhere and apply pressure to the dorsal surface of the forearm, and the second part 3 is adapted to adhere and apply pressure to the surface of the forearm from the inside of the hand. The first part 1 and the second part 3 can be adjusted relative to each other.

In the embodiment, the orthosis has an air flow adjustment system. To this end, the shell of the orthosis is not uniform but has a system of holes 4, preferably on the greatest possible surface area. Preferably, the holes 4 are circular and have a diameter from 3 mm to 25 mm. The shape and arrangement of the holes may be also any other, e.g. in the form of a honeycomb or truss. The system of holes 4 also influences the characteristics of the orthosis itself, i.e. it provides it with adequate flexibility and rigidity.

In order to better adapt the circumference of the orthosis to the patient's forearm, the rigid part of the orthosis, i.e. the body parts (the first part 1 and the second part 3), can be combined with a flexible lining material. The lining material is located on the inner side of the orthosis body, facing the patient's body after it is put on. The lining material may, for example, be made of polyethylene or polyurethane foam.

The clamping pressure on the metacarpal circumference is adjusted by the connection means, for example by Velcro. The orthosis in the embodiment has therefore a Velcro strip 7 and at least one belt loop 5 placed on the outer surface of the first part 1 or the second part 3, preferably on the surface of both these parts. Alternatively, the clamping pressure on the metacarpal circumference can be adjusted with buckles. The buckles for closing the orthosis may have a form known from the prior art, for example analogous to those used in the fasteners of ski boots.

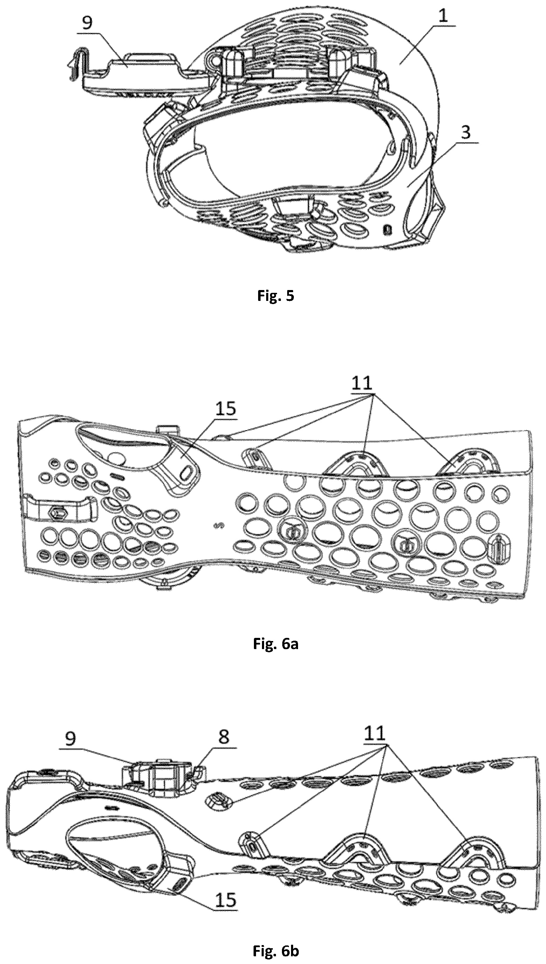

The orthosis can be designed and fabricated in several sizes, so that it is possible to choose a solution tailored to the patient, taking into account the peripheral parameters of the hand. The adjustment system, i.e. the orthosis diameter adjustment unit, enables an efficient adjustment and good selection of the orthosis size. This system is presented in Fig. 1, Fig. 2, and Fig. 3a-3c and comprises the following components: knob 8, cable 10, guides 11 and means for adjusting the position of the first part 1 and the second part 3 in the radial direction, which means comprise guiding surfaces 13 and adjustment locks 12 located on the inside of the first part 1 of the orthosis. The guiding surfaces 13 are sections of the first part 1 between its longitudinal edges and the adjustment locks 12. In other words, the guiding surfaces 13 are sections of the lateral surface of the first part 1 which extend over a few centimetres in width, preferably from 10 mm to 100 mm, and even more preferably from 30 mm to 80 mm. The guiding surfaces 13 of the first part 1 are intended to be connected to the second part 3, i.e. when the two parts are being joined together, the guiding surfaces 13 are slipped over the complementary lateral surfaces of the second part 3. This allows the orthosis to be adjusted in the radial direction, with the minimum diameter of the orthosis cross-section being determined by the adjusting locks 12 located on each of the guiding surfaces 13. The knob 8, to which the cable 10 is attached, is located in a socket located in the wrist area on the first part 1 of the orthosis. The knob 8 is used to adjust the length of the cable 10. The cable 10 which forms the closed loop is fixed in the knob 8 and is passed through the guides 11, preferably located on the sides of the first part 1 of the orthosis, on its outer lateral surfaces along both longitudinal edges. The guides 11 are located on the left and the right side of the first part 1 and, except for those located closest to the knob 8 (after which the cable 10 no longer intersect itself and which are essentially straight), have an arcuate shape (resembling the letter "U") with their open ends (top sections of the letter "U") directed towards the longitudinal edge. Some of the guides 11, located close to the knob 8, may be offset from these edges so as to allow the cable 10 to be directed towards the knob 8 (Fig. 6a, 6b). The guides 11 have a groove or furrow for the cable 10. The cable 10 is passed through these guides 11 so that it passes alternately through the guide 11 located on the left and through the guide 11 located on the right side of the first part 1, so that the relevant sections of the cable 10 intersect in the area of the second part 3, approximately in the middle of the second part 3. Thanks to this arrangement, it is possible to adjust the length of the cable 10 by turning the knob 8 and thus tighten the circumference of the orthosis. In the embodiment, the orthosis has six guides 11, three on each of the two lateral surfaces. To allow the orthosis to be put on, the knob 8 is turned counter clockwise until a distinct click is heard to release the cable 10. The maximum amount of the cable 10 is then released so as to insert the patient's hand inside. When turning the knob 8 clockwise, the cable 10 slides into the mechanism of the knob 8, tightening around the orthosis, so that its components can be perfectly aligned with the forearm. The adjustment of the orthosis size in the radial direction is performed within the width of the guiding surfaces 13 - the maximum tightening of the orthosis with the cable 10 is achieved when the longitudinal edge of the second part 3 contacts the adjustment locks 12. This system allows for possible adjustments to the fitting at a later stage of patient recovery.

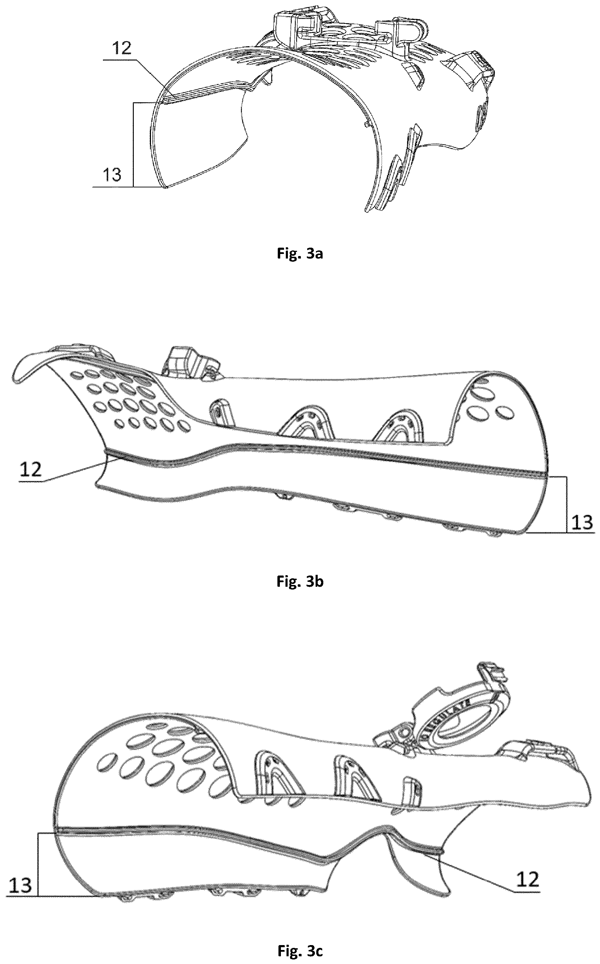

The adjustment locks 12, shown in Fig. 3a-3c, as already mentioned, prevent further adjustment in a given size group and are located on the inside of the first part 1 at a certain distance from the longitudinal edges. The location of the adjustment locks 12 is selected according to the acquired data on the circumferential parameters of the forearm. The adjustment locks 12 in the embodiment are in the form of bulges extending over the entire length of the orthosis along the longitudinal edges, i.e. in the axial direction.

In other embodiments, the adjustment locks 12 may be in a different form, as long as they are fit for purpose. For example, they may be in the form of point protrusions that extend axially along the orthosis and are spaced apart. They can also extend only on a section of the first part 1 adapted to embrace the forearm, except for the palmar part.

A more accurate fit of the orthosis components to the patient's forearm can be achieved by reducing the thickness of the lateral surfaces of the first part 1 forming the guiding surfaces 13. The guiding surfaces 13 in the embodiment are 25% thinner than the central area of this part 1. In other embodiments, the guiding surfaces 13 can be 15-45% thinner than the central area. This increases the flexibility of the first part 1 of the orthosis.

In the embodiment, the orthosis is also fitted with a safety mechanism 9 shown in Fig. 1, securing the knob 8 against unwanted misadjustment. The safety mechanism 9 is in the form of a cap pressed against the knob 8 so as to prevent the cable 10 from being unrolled.

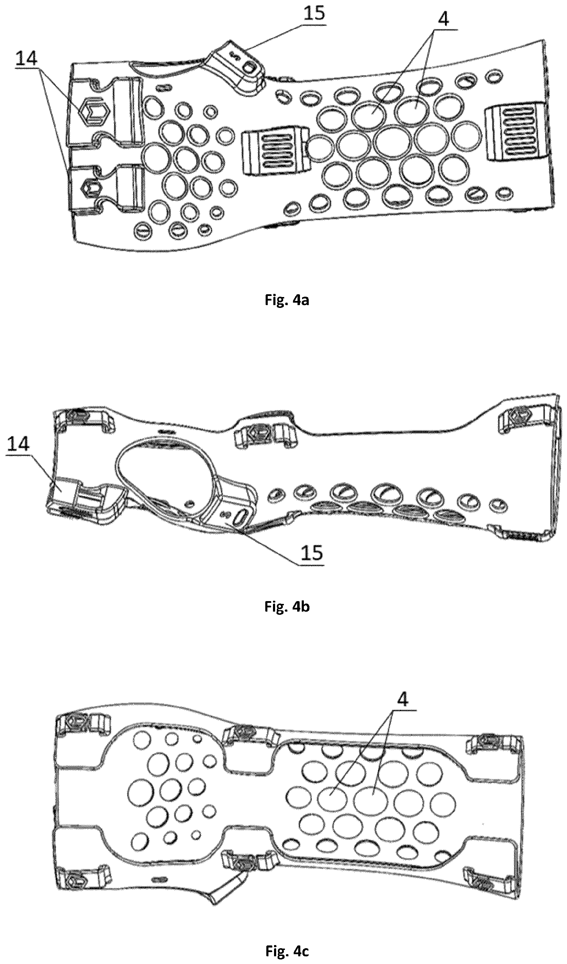

Fig. 4a- 4c show the second part 3 of the orthosis in the form of an orthopaedic splint in the outside, side, and inside view, respectively. Fig. 5 shows the orthosis in the assembled state in the front view, with hand opening visible, while Fig. 6a, 6b show the orthosis in the assembled state in the view from the second part 3 and in the side view, respectively.

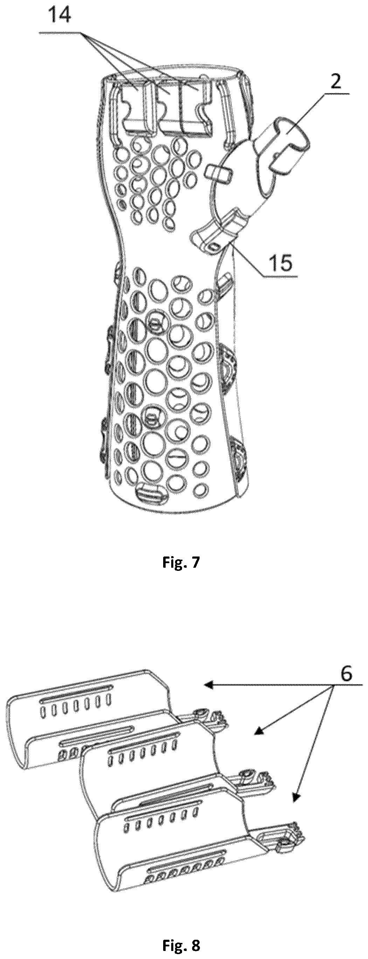

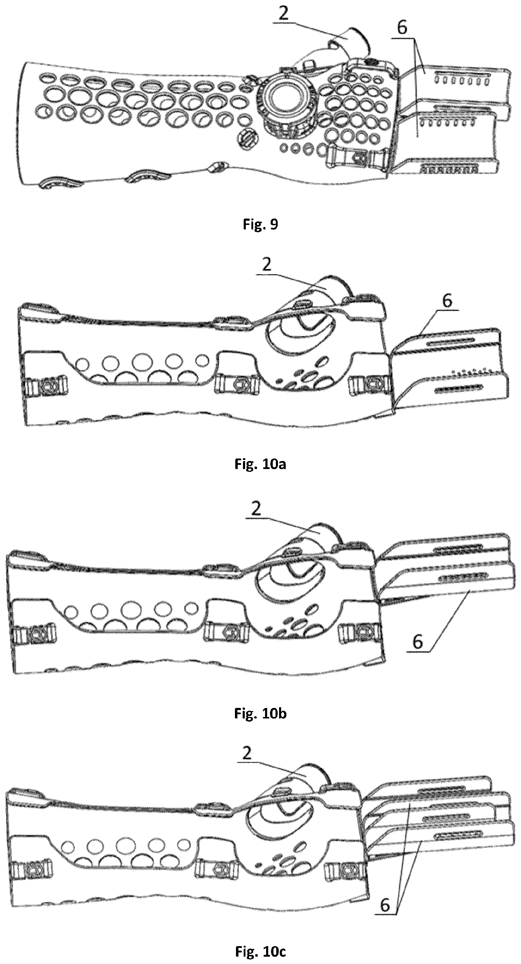

In order to be able to treat injuries involving fingers, the orthosis in the second part 3 comprises at least one socket 14, 15 for accepting at least one finger clip 6, 2 as shown in Fig. 7. In one of the embodiments, the orthosis may comprise the first socket 15 for installation of the thumb clip 2 that stabilizes the thumb. Fig. 6a, 6b show the orthosis with the first socket 15 for the thumb clip 2 (without the clip 2 installed in the orthosis). The thumb clip 2 is shown for example in Fig. 1, Fig. 2, and Fig. 7. For the purpose of treating injuries of other fingers, the orthosis can also be equipped with a system for installation of clips for the remaining fingers 6, intended for individual fingers (other than the thumb) - in Fig. 7 further sockets 14 for clips for fingers other than the thumb are shown. Thus, optionally, the orthosis is provided with a thumb clip 2 (preferably with lining material) to protect the patient's thumb, which clip 2 is inserted in the first socket 15, and/or with further sockets 14, intended for inserting the clips for the other fingers 6. The sockets 14, 15 of the clips 6,2 are located in the second part 3 of the orthopaedic orthosis. The clips for the other fingers 6, which are inserted in the sockets 14, are shown in Fig. 8, while Fig. 9 shows the orthosis with two clips for the other fingers 6 installed. Fig. 10a-10c show the second part 3 of the orthosis in the form of an orthopaedic splint with installed clips for the other fingers, wherein in Fig. 10a one clip 6 for the small and cordial fingers is shown, in Fig. 10b one clip 6 for the central and index fingers is shown, and in Fig. 10c two above clips 6 are shown. Finger mobility can be blocked by using a Velcro strap entwining the clip with a finger placed in it.

Depending on what is required, the design of the orthosis can take the form for positioning the forearm in the neutral position and in the *functional cast* (FC) position. The designation "FC type orthosis" is derived from a plastering method called *"Functional cast bracing".* It is a method in which the forearm positioning consists in dorsal wrist flexion and ulnar deviation.

The orthosis helps to fix the radiocarpal joint in a neutral position and protects against dangerous hyperextensions, flexions and lateral mobility. It effectively reduces the mobility of the wrist, thus reducing the already existing pain associated with inflammation, previous trauma and promotes speeding up of repair processes in the damaged joint structures. The neutral orthosis provides protection to bone cracks that do not require plaster casts, healing after removing the plaster (forearm, wrist, metacarpus), and dislocations of the wrist joint. It is also used after past injuries of the forearm and hand treated conservatively and surgically, as well as after surgical treatment of the forearm and hand for medical reasons, as well as for tendon injuries and inflammations, in the treatment of wrist pain syndromes, acute or chronic inflammations of the wrist or metacarpal bones and after other surgical procedures (orthopaedics, rheumatology) and in neurological diseases of central or peripheral origin.

Further, by making use of the thumb clip 2 the orthosis protects scaphoid fractures or tendinitis within the thumb. By using clips for the other fingers 6, it provides protection for fractures within the metacarpus and phalanges of the fingers as well as for tendinitis. Due to modular design, one orthosis framework provides therefore the treatment of multiple injuries.

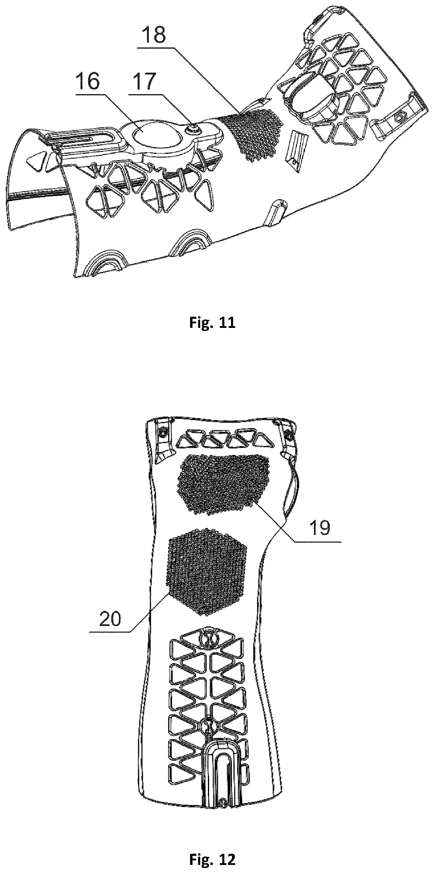

For the purpose of stiffening and stabilizing injuries/fractures of the forearm in the FC or neutral positions, the orthosis is fitted with a unit that enables the prevention of bone displacements in the form of a three-point cushion support system, which allows for the elimination of gaps resulting from (primarily) changes in the size of swelling that occur in during the patient's treatment process. The cushion mounting system is shown in Fig. 11 and Fig. 12, and forms a 3-point fracture support system that is adjustable depending on the type of patient injury. By installing the cushion system, appropriate clamping pressure can be applied to the patient's forearm, wrist, and hand. In addition, the orthosis adjustment system, based on the guides 11, ensures even clamping pressure of the orthosis against the patient's body. Therefore, depending on the swelling of the forearm, the solution allows to easily control the clamping pressure of the orthosis against the patient's forearm. The inflated components (cushions) included in the orthosis are placed at places ensuring full stabilization and preventing the possible movements of the damaged bone-joint structure. They include the first space for the cushion 18, located in the first part 1, near the wrist area (Fig. 11), the second space for the cushion 19, located in the second part 3, near the hand area, and third space for the cushion 20, located in the second part 3, near the wrist area (Fig. 12).

The cushion inflation device shown in Fig. 11 consists essentially of the inflation bulb (pump 16) and the cushions (not shown), which are placed under the orthosis lining material. The pump 16 is made of a flexible material. The deflection of the pump 16 forces the air to flow through the sealed tubing towards the cushions located on the second part 3 and the first part 1 of the orthosis. By adjusting the number of compressions, the volume of the cushions, and therefore the level of filling the space between the patient's forearm and hand and the actual surface of the orthosis is controlled. The inflation device is also fitted with a one-way valve 17 to prevent unwanted air backflow, which at the same time enables the user of the orthosis to fully control the level of filling of the cushions.

Each cushion, in a properly selected shape, is made of, for example, PVC. Each cushion has a valve connected to a non-return valve through which the cushion is supplied with the air. The cushion is inflated, for example, by a pump with a ball valve. The filling of the orthosis cushion is controlled by delivering a sufficient amount of air with the use of the pump 16.

The orthosis has therefore a modular structure, i.e. it has a system of interchangeable components (finger clips, cushions) that can be attached to it, depending on the type of injury and the patient's anatomy. Such a structure makes it versatile, allowing for functionality customization depending on the nature of the individual forearm injury.

The orthosis itself, i.e. its shell, can be fabricated from, for example, PA12 polyamide and 3D printed, in the Selective Laser Sintering or HP Multijet Fusion technology. Other suitable materials for the fabrication of the orthosis are, e.g. PLA, ABS, Nylon 6 or PETg.

The orthosis may be also provided with a protection against water absorption in the form of a skin-safe protective coating applied to it. The orthosis can also be coloured to achieve the desired colour.

## Claims

1. An orthopaedic orthosis, especially for upper limb, comprising
a body made of a first part (1) and a second part (3) adapted for being joined together around the limb to be stabilized, the first part (1) and the second part (3) being adjustable relative to each other;
connection means for joining together the first part (1) and the second part (3); and
an adjustment system for adjusting the position of the first part (1) and the second part (3) relative to each other, **characterised in that**
the adjustment system comprises means for adjusting the position of the first part (1) and the second part (3) in the radial direction, said means include guiding surfaces (13) disposed on the first part (1) and adjustment locks (12),
wherein the adjustment locks (12) are disposed on the inside of the first part (1), extend in the axial direction of the orthosis and determine the minimum cross-section of the orthosis in the radial direction,
and wherein the guiding surfaces (13) constitute sections of the first part (1) extending between its longitudinal edges and these adjustment locks (12), whereby the guiding surfaces (13) are slipped over the complementary lateral surfaces of the second part (3) while the first part (1) and the second part (3) are being joined together.

2. The orthopaedic orthosis according to claim 1, **wherein** the guiding surfaces (13) can be from 1 cm to 10 cm in width, more preferably from 3 cm to 8 cm.

3. The orthopaedic orthosis according to claim 1 or 2, **wherein** the means for adjusting the position of the first part (1) and the second part (3) comprise additionally a cable (10) attached to a knob (8) disposed on the first part (1), and guides (11) disposed alternately on the left and the right side of the first part (1) on its lateral surfaces along both longitudinal edges, wherein the cable (10) forms a closed loop and is passed through the guides (11) so that it passes alternately through the guides (11) disposed on the left and the right side of the first part (1), so that the individual cable (10) sections intersect in the area of the second part (3).

4. The orthopaedic orthosis according to claim 1 or 2 or 3, **wherein that** the second part (3) further comprises at least one socket (14, 15) for receiving at least one finger clip (6, 2).

5. The orthopaedic orthosis according to claim 4, **wherein** it comprises a thumb clip (2) inserted into the socket (15).

6. The orthopaedic orthosis according to either claim 4 or 5, **wherein** it comprises at least one clip for the other fingers (6) inserted into the socket (14).

7. The orthopaedic orthosis according to any claim 1 to 6, **wherein** the adjustment locks (12) are in the form of continuous bulging or spot protrusions extending along the longitudinal edge and at a certain distance from it.

8. The orthopaedic orthosis according to any claim 1 to 7, **wherein** the first part (1) and the second part (3) are fitted with lining material on their inner sides.

9. The orthopaedic orthosis according to any claim 1 to 8, **wherein** it comprises a safety mechanism (9) that protects the knob (8) from misadjustment, preferably in the form of a latch.

10. The orthopaedic orthosis according to any claim 1 to 9, **wherein** it has six guides (11), three on each of the lateral surfaces of the first part (1).

11. The orthopaedic orthosis according to any claim 1 to 10, **wherein** the guiding surfaces (13) are 15-45% thinner than the central section of the first part (1), extending between the adjustment locks (12).

12. The orthopaedic orthosis according to any claim 1 to 11, **wherein** it has spaces for installation of cushions, wherein the first cushion space (18) is located in the first part (1), the second cushion space (19) is located in the second part (3) near the hand area, and the third cushion space (20) is located in the second part (3) near the wrist area.

13. The orthopaedic orthosis according to claim 12, **wherein** it has cushions installed in the first cushion space (18), in the second cushion space (19) and in the third cushion space (20).

14. The orthopaedic orthosis according to either claim 12 or 13, **wherein** it has a cushion inflation device, comprising a pump (16) made of a flexible material and tightly connected by tubing with each of the cushions, wherein each of the cushions is fitted with a valve.

15. The orthopaedic orthosis according to claim 14, **wherein** the cushion inflation device is fitted with a one-way valve (17) preventing the air from flowing back from the cushions.
